# EUROPEAN PATENT SPECIFICATION

(11) **EP 1 656 358 B1**
(45) Date of publication and mention of the grant of the patent: **23.02.2011**
(21) Application number: 04766517.9
(22) Date of filing: 18.08.2004
(51) Int. Cl.: C07D 263/58, A61K 31/42, A61P 25/16

(54) **STABLE POLYMORPH OF BIFEPRUNOX MESILATE**
STABILER POLYMORPH VON BIFEPRUNOXMESILAT
POLYMORPHE STABLES DU MESILATE DE BIFEPRUNOX

(30) Priority: 18.08.2003 EP 03102573; 18.08.2003 US 495708 P
(43) Date of publication of application: 17.05.2006
(73) Proprietor: Abbott Healthcare Products B.V., 1381 CP Weesp (NL)
(72) Inventor: ZWIER, Klaas, NL-1381 CP Weesp (NL); KLEIN, Gerrit, NL-1381 CP Weesp (NL); EIJGENDAAL, Irene, NL-1381 CP Weesp (NL); TER HORST-VAN AMSTEL, Maria, J.L., NL-1381 CP Weesp (NL)
(74) Representative: Verhage, Marinus
(86) International application number: PCT/EP2004/051816
(87) International publication number: WO 2005/016898

(56) References cited:
- WO-A-02/066449
- HALEBLIAN J ET AL: "PHARMACEUTICAL APPLICATIONS OF POLYMORPHISM" JOURNAL OF PHARMACEUTICAL SCIENCES, AMERICAN PHARMACEUTICAL ASSOCIATION. WASHINGTON, US, vol. 58, no. 8, 1 August 1969 (1969-08-01), pages 911-929, XP002020518 ISSN: 0022-3549

## Description

The present invention relates to a stable polymorphic form of the compound 7-[4-([1,1'-biphenyl]-3-ylmethyl)-1-piperazinyl]-2(3H)-benzoxazolone monomethanesulfonate, a method for the preparation of said polymorphic form and its use in pharmaceutical products, especially in pharmaceutical products for the treatment of psychotic disorders and Parkinson's disease.

The mesylate of the compound 7-[4-([1,1'-biphenyl]-3-ylmethyl)-1-piperazinyl]-2(3H)-benzoxazolone monomethanesulfonate (INNM bifeprunox mesilate) has the formula

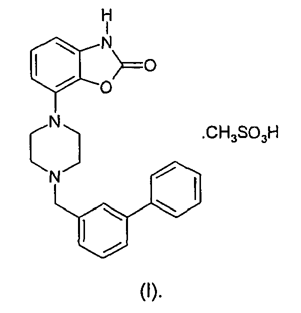

The hydrochloric acid salt of this compound (7-[4,([1,1',biphenyl],3,ylmethyl),1, piperazinyl]-2(3H)-benzoxazolone (bifeprunox) is described and claimed in WO97/36893 and the monomethanesulfonate salt is described and claimed in WO02/066449. In the second patent publication the direct formation of the monomethanesulfonate salt by the reaction between the reactive mesylate ester of N,N,N-bis(2-ethanol)-m-phenylbenzyl amine and 7-amino-2(3H)- benzoxazolone is disclosed.

The inventors have now found that by the method described in WO02/066449 bifeprunox mesylate is normally obtained as a crude product (melting range indicated in WO02/066 as 263 - 275 °C) in a polymorphic form further indicated in this application as polymorph δ (delta). Upon further purification the product is obtained in two different crystal modifications or a mixture of these two modifications. The first of said two modifications is the already mentioned polymorph δ (delta) and has a melting point in pure form of 265 °C. The second modification is further indicated as polymorph γ (gamma). When the γ polymorph is predominantly is obtained, it is in almost all cases obtained in a mixture of said polymorph with polymorph δ, the mixture having a melting point of about 273 °C.

During further investigations it has appeared that pol ymorphs γ and δ are metastable, and therefore may have serious drawbacks when used in a pharmaceutical formulation. The unpredictable formation of one of the two polymorphs γ and δ or a mixture thereof is also undesirable. It is therefore the object of the present invention to provide a stable crystalline form of 7-[4-([1,1'-biphenyl]-3-ylmethyl)-1-piperazinyl]-2(3H)-benzoxazolone monomethanesulfonate for pharmaceutical use which can be produced in a reproducible manner.

It has now surprisingly been found that 7-[4-([1,1'-biphenyl]-3-ylmethyl)-1-piperazinyl]-2(3H)-benzoxazolone monomethanesulfonate also has another crystalline polymorphic form (referred to below as polymorph α (alpha)) which does not have the disadvantages of the earlier mentioned polymorphs. This crystalline form of bifeprunox mesylate is more thermodynamically stable. Polymorphic form α does not undergo conversion, even at high atmospheric humidity or higher temperature. Furthermore this crystalline form crystallizes in the form of large crystals which can be easily be filtrated and having a high purity. Therefore this polymorph α is particularly suitable for the formulation of bifeprunox mesylate in a solid form, if desired after particle size reduction.

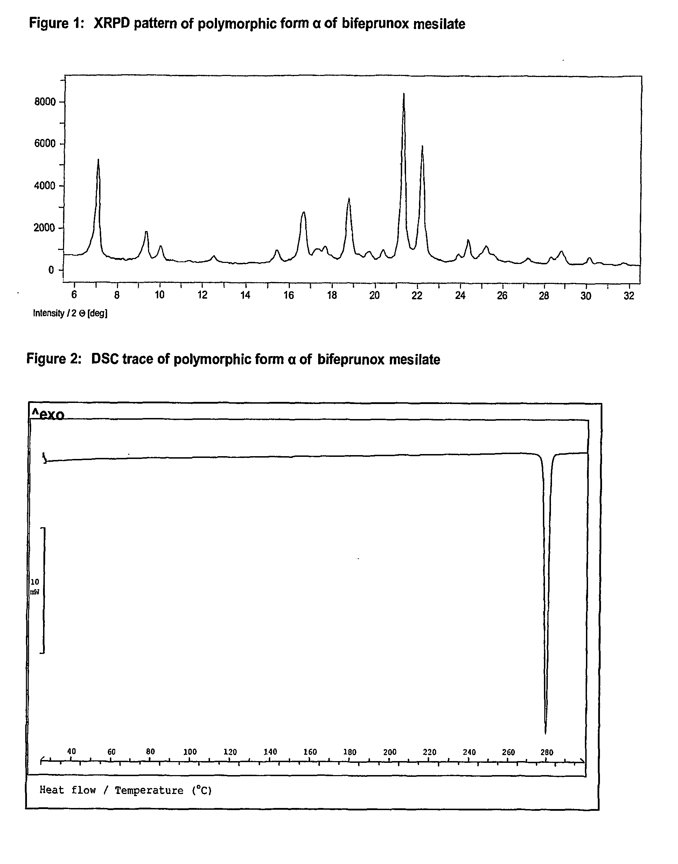

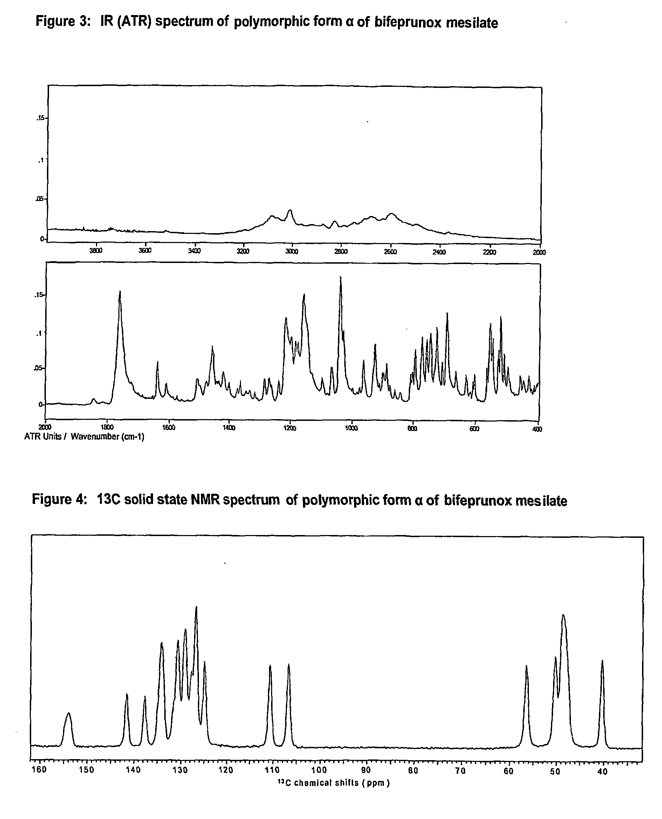

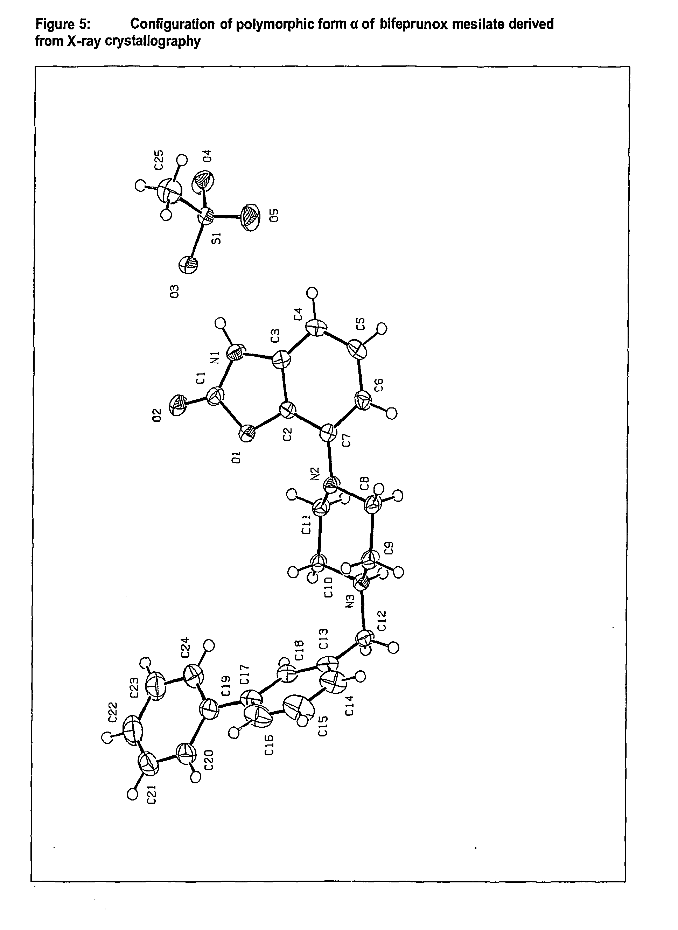

The crystalline polymorphic form of bifeprunox mesylate according to the present invention is defined by the following physicochemical parameters
X-Ray diffraction patterns (Table 1 and Figure 1);
The melting point of polymorphic form α is 277 °C (DSC heating rate 10 K/min) (see DSC thermogram, Figure 2);
IR spectrum (Table 2 and Figure 3); The most important IR absorption bands of form α of bifeprunox mesylate which can be used to distinguish this form from forms γ and γ are given in Table 2a;
Solid state ¹³C-NMR spectrum (Table 3 and Figure 4); The most important ¹³C-NMR shifts of form α of bifeprunox mesylate which can be used to distinguish this form from forms γ and δ are given in Table 3a;
Single crystal X-ray diffraction (Tables 4 and 5 and Figure 5).

All data herein are understood to be approximate and subject to normal measurement error depending e.g. on the apparatus used and other parameters influencing peak positions and peak intensities.

**Table 1. Characteristic Powder X-ray Diffractions of forms α, γ and δ of bifeprunox mesylate. Figure 1 provides a representative XRPD pattern of polymorphic form α of bifeprunox mesylate.**

| Form | Characteristic reflexes (expressed in degree of diffraction angle 2θ at room temperature) |
|---|---|
| α | 7.0, 9.3, 10.0, 12.5, 15.4, 16.7, 17.2, 17.4, 17.7, 18.7, 21.3, 22.2, 25.2, 27.2, 28.3, 28.8, 30.1 |
| γ | 10.4, 11.4, 11.7, 14.1, 15.1, 21.0, 26.9 |
| δ | 6.4, 10.2, 12.1, 16.4, 16.8, 19.3, 19.7, 20.6, 24.1, 26.6 |

**Table 2. Characteristic IR absorption bands of forms α, γ and δ of bifeprunox mesylate. Figure 2 provides a representative IR spectrum of polymorphic form α of bifeprunox mesylate**

| Form | Characteristic IR absorption bands (expressed in cm⁻¹) |
|---|---|
| α | 1764, 1636, 1284, 1217, 809, 795, 746, 694, 663, 509 |
| δ | 1777, 1279, 1258, 1210, 1124, 800, 764, 749, 627, 518 |
| δ | 1865, 1769, 1434, 1282, 1253, 1212, 1126, 935, 767, 751 |

**Table 2a. Most important IR absorption bands of forms α, γ and δ of bifeprunox mesylate which can be used to distinguish the three forms.**

| Form | Characteristic IR absorption bands (expressed in cm⁻¹) |
|---|---|
| α | 1764,1217,795,746,694 |
| γ | 1777, 1210, 764, 749, 518 |
| δ | 1769, 1212, 935, 767, 751 |

**Table 3. Characteristic ¹³C solid state NMR chemical shifts in forms α, γ and δ of bifeprunox mesylate. Figure 3 provides a representative ¹³C solid state NMR spectrum of polymorphic form α of bifeprunox mesylate**

| Form | Characteristic chemical shift (expressed in ppm relative to glycine (δ_{c} =176.03 for the C=O resonance) |
|---|---|
| α | 40.4, 48.7, 50.3, 56.5, 106.8, 110.7, 124.9, 126.9, 127.8, 129.2, 130.8, 134.2, 137.7, 141.6, and *153.8. |
| γ | 38.2, *44.3, *45.9, 50.1, 54.5, 59.4, 103.5, 109.3, 125.3, 127.9, 128.9, 131.1, 133.2, 134.5, 141.2, 143.2 and *153.7 |
| δ | 39.1, *44.3, *46.3, 49.3, 53.4, 58.8, 104.6, 110.4, 124.6, 127.0, 128.5, 129.7, 130.5, 134.4, 141.5, 143.5, and *154.7 |

| | |
|---|---|
| *) denotes carbon resonances which show typical asymmetric residual quadrupolar splittings. Chemical shift are given for the high -field resonance maximum | |

**Table 3a. Most important ¹³C solid state NMR chemical shifts bands of forms α, γ and δ of bifeprunox mesylate which can be used to distinguish the three forms.**

| Form | Characteristic chemical shift (expressed in ppm relative to glycine (δc =176.03 for the C=O resonance) |
|---|---|
| α | 40.4, 48.7, 56.5, 106.8 and 137.7 |
| γ | 38.2, 54.5,103.5, 109.3 and 133.2 |
| δ | 39.1, 49.3, 53.4, 58.8 and 104.6 |

**Table 4. Relevant Single Crystal X-ray Diffraction data collection parameters for the crystal structure determination of forms α, γ and δ of bifeprunox mesylate.**

| | Alpha (α) | Gamma (γ) | Delta (δ) |
|---|---|---|---|
| Temperature (K) | 150 | 133 | 150 |
| Wavelength (A) | 0.71073¹ | 0.71073 | 0.71073 |
| Crystal size (mmxmmxmm) | 0.10x0.15x0.27 | 0.24x.13x0.07 | 0.10x0.15x0.35 |
| Crystal system | triclinic | monoclinic | triclinic |
| Space group | P-1 | P2₁/c | P-1 |
| Z | 2 | 4 | 2 |
| Unit cell dimensions;a (A) | 9.823 | 9.0975 | 9.1832 |
| B (Å) | 10.737 | 15.269 | 9.3963 |
| C (Å) | 12.690 | 17.128 | 14.106 |
| a (°) | 98.553 | 90 | 76.968 |
| β (°) | 93.749 | 100.694 | 83.809 |
| Γ (°) | 116.097 | 90 | 189.157 |
| Calculated density (g cm⁻³) | 1.481 | 1.368 | 1.3556 |
| Completeness of data (%) | 100.0 | 100.0 | 99.8 |
| Total number of reflections | 27105 | 23759 | 27207 |
| Number of unique reflections | 5355 | 5809 | 4149 |
| Nr. Of refined parameters | 314 | 316 | 314 |

| | | | |
|---|---|---|---|
| (Mo Kα radiation) | | | |

**Table 5. Atomic coordinates (x 10⁴) and equivalent isotropic displacement parameters (A²x 103) of crystal structure of form α of bifeprunox mesylate. U(eq) is defined as one third of the trace of the orthogonalized Uᵢⱼ tensor.**

| | x | y | z | U(eq) |
|---|---|---|---|---|
| O1 | 3471.7(11) | 3848.0(10) | 2910.8(8) | 26.4(3) |
| 02 | 2785.8(13) | 1499.8(11) | 2541.1(10) | 38.1(4) |
| N1 | 5215.5(15) | 3175.4(14) | 3398.0(11) | 29.3(4) |
| N2 | 3880.6(13) | 6773.4(13) | 3211.0(10) | 24.6(4) |
| N3 | 1702.0(14) | 7879.1(13) | 3177.7(11) | 24.9(4) |
| C1 | 3755.9(18) | 2687.4(17) | 2914.9(13) | 28.6(5) |
| C2 | 4801.7(16) | 5042.8(16) | 3421.0(12) | 23.7(4) |
| C3 | 5896.6(17) | 4637.6(16) | 3727.8(12) | 25.8(5) |
| C4 | 7334.0(17) | 5622.3(17) | 4265.8(12) | 28.3(5) |
| C5 | 7587.8(18) | 7016.7(18) | 4470.3(12) | 30.7(5) |
| C6 | 6489.3(17) | 7425.7(17) | 4145.1(12) | 28.2(5) |
| C7 | 5035.3(17) | 6432.4(16) | 3594.8(12) | 24.3(4) |
| C8 | 4371.2(18) | 8285.6(16) | 3280.4(14) | 29.8(5) |
| C9 | 3141.4(17) | 8515.3(17) | 2694.6(13) | 29.2(5) |
| C10 | 1196.3(17) | 6328.4(15) | 3094.7(13) | 25.8(5) |
| C11 | 2450.2(16) | 6106.0(16) | 3661.4(12) | 25.9(5) |
| C12 | 465.7(18) | 8238.1(17) | 2763.9(13) | 29.1(5) |
| C13 | -273.5(18) | 7526.6(18) | 1622.4(13) | 30.9(5) |
| C14 | 166(2) | 8245(2) | 780.4(15) | 46.7(7) |
| C15 | -586(2) | 7574(3) | -256.4(16) | 57.6(8) |
| C16 | -1734(2) | 6194(2) | -466.0(15) | 49.2(7) |
| C17 | -2206.8(19) | 5456.1(19) | 362.2(13) | 34.9(6) |
| C18 | -1474.4(18) | 6157.3(18) | 1409.5(13) | 30.8(5) |
| C19 | -3495(2) | 4003.7(19) | 170.3(13) | 37.1(6) |
| C20 | -4751(2) | 3585(2) | -623.3(14) | 43.7(6) |
| C21 | -5976(2) | 2260(2) | -766.8(17) | 54.6(7) |
| C22 | -5989(2) | 1318(2) | -129.3(18) | 58.2(8) |
| C23 | -4750(3) | 1709(2) | 655.0(17) | 54.6(7) |
| C24 | -3520(2) | 3039(2) | 804.2(15) | 45.3(6) |
| S1 | 8220.4(4) | 1865.1(4) | 3801.4(3) | 26.8(1) |
| O3 | 6650.8(13) | 1454.8(12) | 3355.9(10) | 40.2(4) |
| O4 | 8282.1(15) | 1197.6(13) | 4711.2(9) | 42.6(4) |
| O5 | 9171.5(14) | 3369.6(12) | 4040.9(11) | 48.7(4) |
| C25 | 8951(2) | 1114(2) | 2801.2(15) | 51.0(7) |

**Table 6. Atomic coordinates (x 10⁴) and equivalent isotropic displacement parameters (Å²x 10³) of crystal structure of form of bifeprunox mesylate. U(eq) is defined as one third of the trace of the orthogonalized Ups tensor.**

| | x | y | z | U(eq) |
|---|---|---|---|---|
| O(1) | 6610.7(11) | 756.7(7) | 6306.4(6) | 26.6(2) |
| O(2) | 9117.5(12) | 552.8(8) | 6513.4(8) | 40.3(3) |
| C(2) | 7882.4(17) | 240.9(11) | 6352.9(10) | 29.1(4) |
| N(3) | 7439.1(14) | -598.9(9) | 6206.3(8) | 27.4(3) |
| C(3A) | 5878.6(17) | -646.4(10) | 6063.5(9) | 24.5(3) |
| C(4) | 4896.5(18) | -1346.6(11) | 5948.7(9) | 31.7(4) |
| C(5) | 3392.7(19) | -1133.4(11) | 5866.9(10) | 35.5(4) |
| C(6) | 2894.2(18) | -281.1(11) | 5915.0(9) | 32.3(4) |
| C(7) | 3884.2(17) | 428.8(10) | 6069.0(9) | 26.2(3) |
| C(7A) | 5382.3(16) | 199.5(10) | 6119.9(8) | 23.7(3) |
| N(1') | 3465.6(14) | 1286.2(8) | 6230.9(8) | 28.4(3) |
| C(2') | 1876.4(18) | 1434.5(11) | 6215.9(11) | 35.7(4) |
| C(3') | 1661.2(18) | 2283.3(11) | 6630.8(11) | 36.5(4) |
| N(4') | 2322.4(14) | 3039.8(9) | 6262.9(8) | 28.0(3) |
| C(5') | 3942.4(17) | 2861.2(11) | 6265.4(10) | 30.0(4) |
| C(6') | 4103.6(17) | 2010.2(10) | 5840.3(9) | 27.2(3) |
| C(10) | 2051(2) | 3884.9(11) | 6667.0(10) | 35.6(4) |
| C(11) | 2788.0(18) | 4658.9(11) | 6354.3(9) | 30.7(4) |
| C(12) | 2314.0(17) | 4949.2(10) | 5577.8(9) | 27.8(4) |
| C(13) | 3015.0(17) | 5646.9(10) | 5277.0(9) | 26.8(3) |
| C(14) | 4183.8(18) | 6072.6(11) | 5781.3(10) | 33.7(4) |
| C(15) | 4644(2) | 5795.6(12) | 6554.8(11) | 40.5(4) |
| C(16) | 3964.4(19) | 5086.4(12) | 6836.9(10) | 38.5(4) |
| C(21) | 2576.4(16) | 5917.7(10) | 4432.7(9) | 25.3(3) |
| C(22) | 2266.8(17) | 5286.3(11) | 3836.1(9) | 29.8(4) |
| C(23) | 1921.6(19) | 5532.9(11) | 3043.3(10) | 35.0(4) |
| C(24) | 1900.5(18) | 6409.3(11) | 2833.0(10) | 33.3(4) |
| C(25) | 2200.7(17) | 7041.3(11) | 3419.1(10) | 31.8(4) |
| C(26) | 2519.2(17) | 6797.7(10) | 4209.4(10) | 29.3(4) |
| S | 9163.9(4) | -2786.7(3) | 5975.1(2) | 28.4(1) |
| O(3) | 9584.0(13) | -1870.9(7) | 6067.6(8) | 39.5(3) |
| O(4) | 7714.0(13) | -2961.4(8) | 6156.0(8) | 48.0(4) |
| O(5) | 9327.4(15) | -3123.8(9) | 5197.7(7) | 50.7(4) |
| C(1M) | 10484.0(18) | -3388.0(11) | 6647.3(9) | 33.1(4) |

**Table 7. Atomic coordinates (x 10⁴) and equivalent isotropic displacement parameters (Å²x 10³) of crystal structure of form δ of bifeprunox mesylate. U(eq) is defined as one third of the trace of the orthogonalized Uᵢⱼ tensor.**

| | x | y | z | U(eq) |
|---|---|---|---|---|
| 01 | 4353.9(14) | 2151.8(14) | 9.8(9) | 31.7(5) |
| 02 | 2013.8(15) | 1799.9(16) | -260.0(11) | 41.7(6) |
| N1 | 3473.0(19) | 3697(2) | -1220.7(13) | 33.0(6) |
| N2 | 7357.2(17) | 2103.5(19) | 550.2(12) | 32.1(6) |
| N3 | 8449.3(17) | 325.4(19) | 2265.2(12) | 30.5(6) |
| C1 | 3130(2) | 2515(2) | -491.7(15) | 31.6(8) |
| C2 | 5441(2) | 3184(2) | -431.7(14) | 27.6(7) |
| C3 | 4912(2) | 4146(2) | -1202.7(15) | 29.5(7) |
| C4 | 5792(2) | 5233(2) | -1804.5(16) | 38.0(8) |
| C5 | 7222(2) | 5295(2) | -1576.5(17) | 40.4(8) |
| C6 | 7745(2) | 4328(2) | -796.9(16) | 35.7(8) |
| C7 | 6862(2) | 3203(2) | -190.9(15) | 29.9(7) |
| C8 | 8926(2) | 2107(2) | 662.0(15) | 36.3(8) |
| C9 | 9346(2) | 659(2) | 1284.5(15) | 36.0(7) |
| C10 | 6854(2) | 364(2) | 2127.3(15) | 33.3(7) |
| C11 | 6484(2) | 1826(2) | 1508.8(14) | 34.0(7) |
| C12 | 8900(2) | -1091(2) | 2896.8(15) | 35.7(8) |
| C13 | 7978(2) | -1468(2) | 3868.2(15) | 32.6(7) |
| C14 | 6997(2) | -2644(2) | 4086.9(17) | 40.5(8) |
| C15 | 6109(2) | -2941(2) | 4966.4(17) | 42.7(8) |
| C16 | 6171(2) | -2068(2) | 5624.7(16) | 39.1(8) |
| C17 | 7146(2) | -888(2) | 5437.5(15) | 32.1(7) |
| C18 | 8054(2) | -613(2) | 4552.0(15) | 32.2(7) |
| C19 | 7171(2) | 74(2) | 6137.9(15) | 31.4(7) |
| C20 | 7068(2) | -494(2) | 7144.3(15) | 34.8(7) |
| C21 | 7028(2) | 422(3) | 7794.2(16) | 38.2(8) |
| C22 | 7099(2) | 1919(3) | 7448.1(16) | 39.7(8) |
| C23 | 7201(2) | 2497(2) | 6453.5(16) | 41.0(8) |
| C24 | 7234(2) | 1589(2) | 5798.4(16) | 37.9(8) |
| S1 | 8731.8(6) | 3909.1(6) | 3076.9(4) | 33.3(2) |
| O3 | 9471.7(16) | 2602.8(16) | 2887.4(12) | 50.3(6) |
| O4 | 7233.7(16) | 3640.6(18) | 3484.5(11) | 50.4(6) |
| O5 | 8877.7(16) | 5117.0(17) | 2228.8(11) | 47.6(5) |
| C25 | 9712(3) | 4404(3) | 3958.1(17) | 48.8(9) |

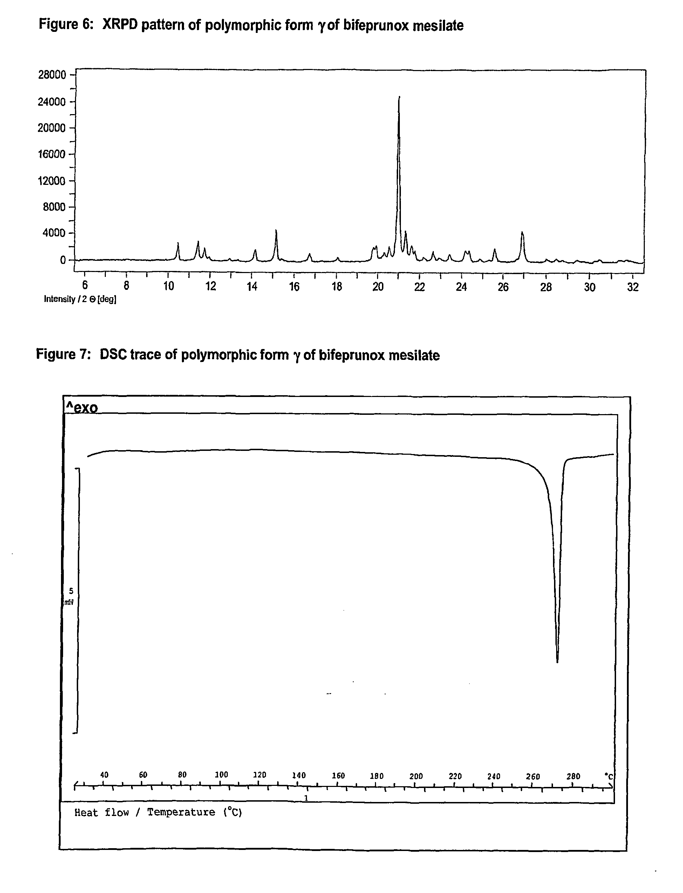

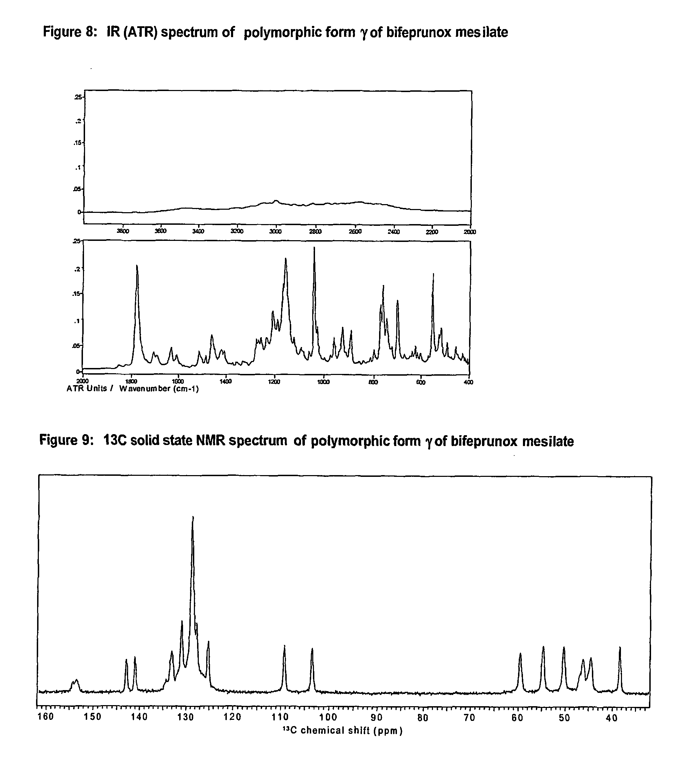

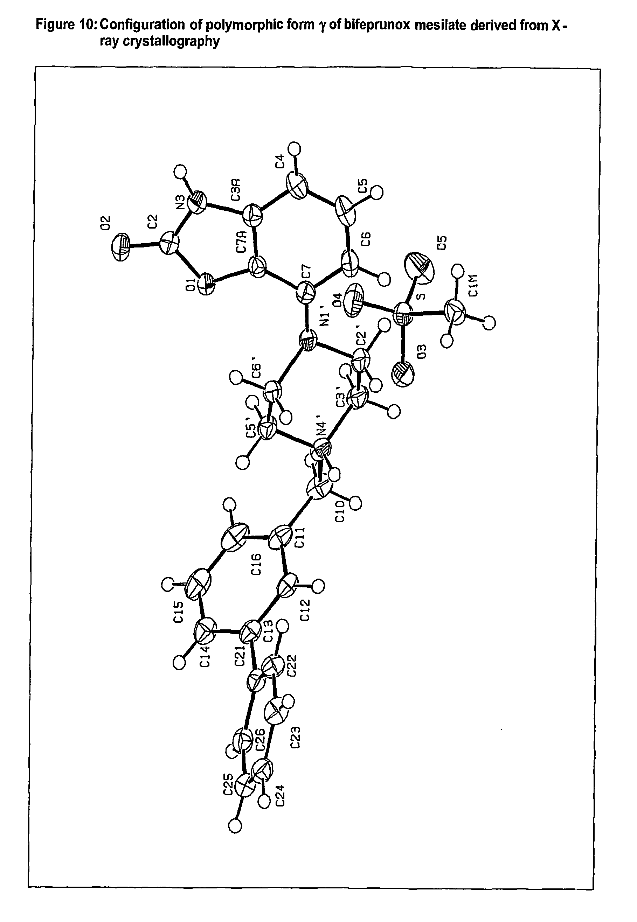

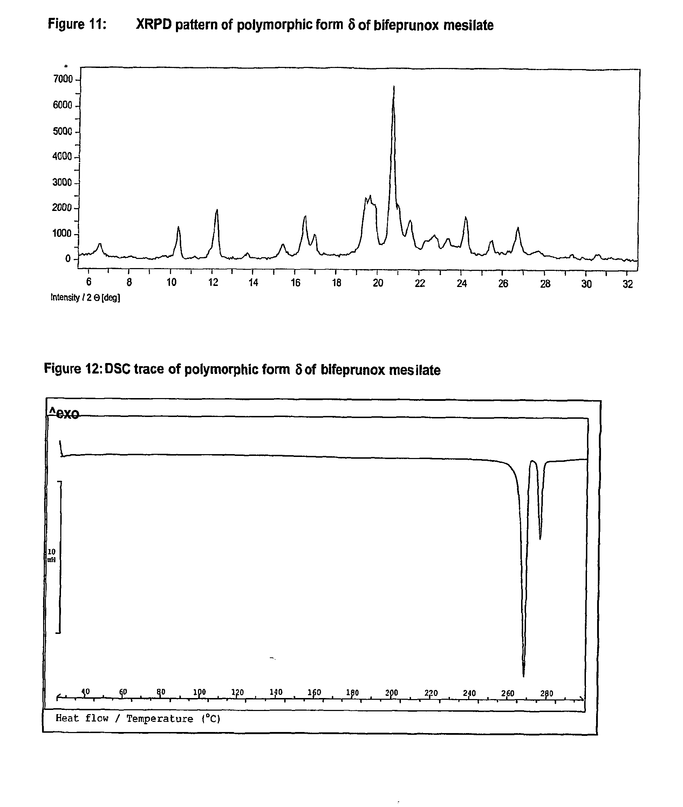

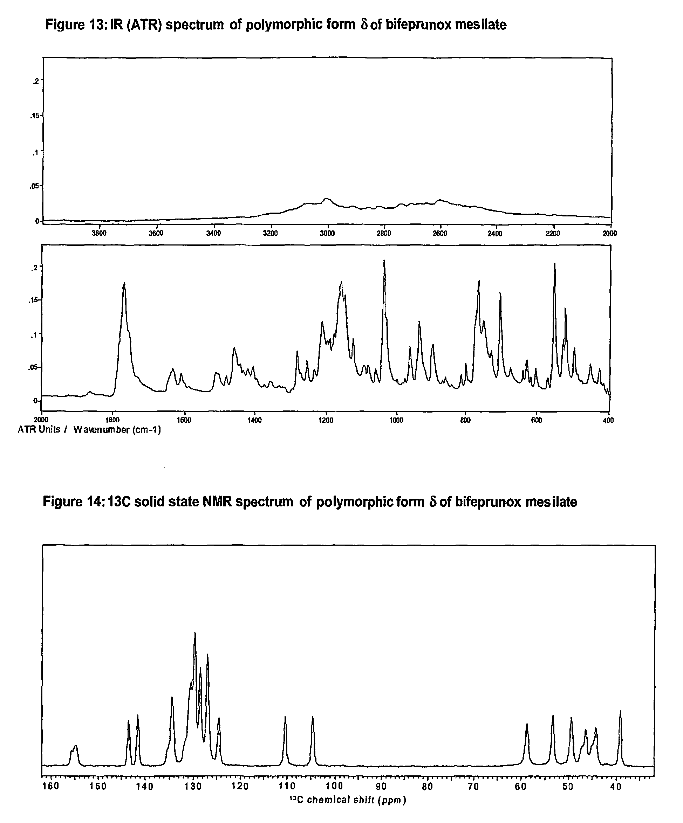

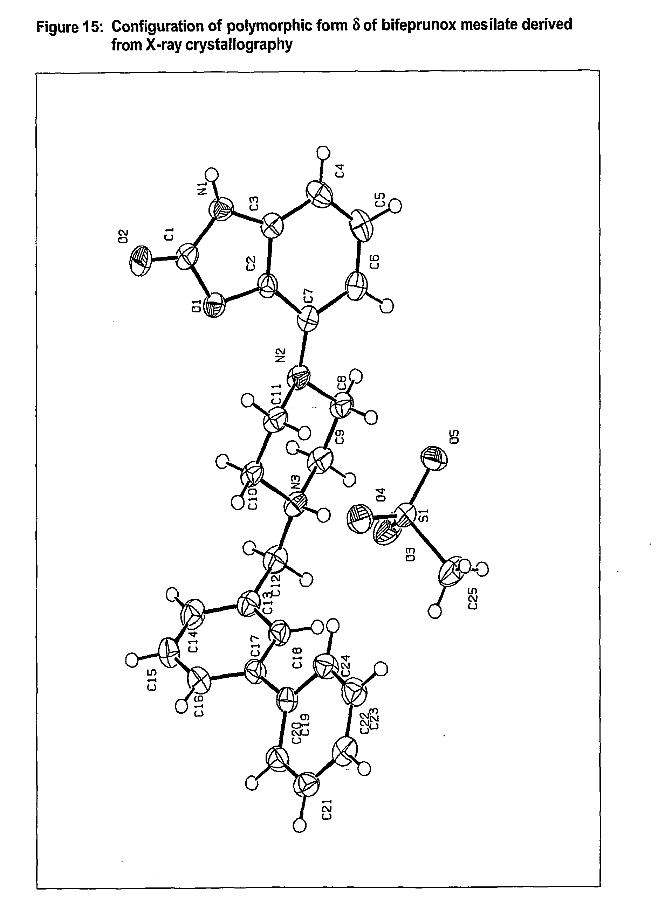

The polymorphic form α differs substantially from the forms γ and δ in its physicochemical parameters: DSC melting behavior, X-ray diffraction pattern, IR spectrum and solid state ¹³C-NMR spectrum. The physicochemical parameter of the forms γ and δ are given in Tables 1-4, 6 and 7 and Figures 6-15.

The present invention also relates to bifep runox mesylate in which at least about 50 weight percent (wt. %) of the bifeprunox mesylate, preferably at least about 60 wt. % thereof, more preferably at least about 80 wt. % thereof, more advantageously, at least about 90 wt. %, yet more preferably at least about 95 wt % of bifeprunox mesylate is in the polymorphic a form and is substantially devoid of any γ or δ polymorphic forms thereof. With substantially devoid is meant an amount of less than 10%, preferably less than 5% w/w. Still more preferably at least a bout 99% wt. % of bifeprunox mesylate is in the polymorphic α form.

The preparation of the polymorphic form a according to the invention is carried out by recrystallisation from an organic solvent or a mixture of an organic solvent with water, preferably a mixture of a (C₁-C₆)alcohol and water or a mixture of acetonitrile and water. More preferred are a mixture of 2-propanol and water or a mixture of acetonitrile and water. The most preferred solvent is a mixture of acetonitrile and water. The polymorphic form γ can be prepared by making the free base of bifeprunox directly followed by the addition of methane sulphonic acid and crystallization from methylethylketone.

The polymorphic form α according to the invention can be formulated into dosage forms in which the crystalline active substance is present in the solid form by methods known in the art. Examples of said dosage forms are (optionally coated) tablets, capsules, granular aerosols, suppositories and suspensions, which can be prepared by mixing the polymorphic form α of the active substance with inert pharmaceutically acceptable excipients and carriers. Most preferably the dosage forms are tablets or capsules.

In a preferred formulation of the polymorphic form s a according to the invention the composition contains, apart from the milled and sieved active substance, lactose monohydrate, microcrystalline cellulose, sodium starch glycolate type A, sodium stearyl fumarate and optionally colloidal anhydrous silica. The amount of lactose is between 20 and 90 % w/w of the total weight of the tablet core, preferably between 70 and 90 % w/w and most preferred between 75 and 85 % w/w. The amount of microcrystalline cellulose is between 5 and 90% w/w of the total weight of the tablet core, preferably between 10 and 15 % w/w and most preferred between 11 and 12 % w/w. The amount of sodium starch glycolate type A is between 0.1 % and 2.5 % w/w of the total weight of the tablet core, preferably between 0.3 and 0.7 % w/w and most preferred about 0.5% w/w. The amount of sodium stearyl fumarate is between 0.1 % and 1.5 % of the total weight of the tablet core, preferably between 0.6 and 1.3 % w/w and most preferred about 1.0% w/w. Collodial anhydrous silica is optionally added to the formulation in order to improve the flow properties of the powder. When added it is preferably added in an amount of between 0.05% and 0.5 % w/w of the total weight of the tablet core, preferably about 0.4% w/w. The amount of coating is between 2.0% and 5.0 % w/w of the weight of the tablet core, preferably between 3.0 and 4.0 % w/w and most preferred about 3.5%.

In a further aspect the invention relates to a method of producing the formulation described above, wherein the active substance having polymorphic form α according to the invention is milled and subsequently mixed with a suitable mixer (e.g. an orbital screw mixer (Nauta mixer) or a combination of a diffusion mixer (bin blender) with a rotating impeller mill (quadro co-mill)) with lactose monohydrate, microcrystalline cellulose, sodium starch glycolate type A, sodium stearyl fumarate and optionally with colloidal anhydrous silica and pressed into tablets of the desired strength. During tabletting the pressure is between 200 and 400 MPa, preferably between 250 and 350 and most preferred about 300 MPa. The product is coated with a colour and taste coating by spraying of a coating suspension onto the tablet core in a suitable coating equipment (e.g. a perforated pan coater or a fluidized bed coater)

The polymorphic form α according to the invention can be used by administering to a living being. Bifeprunox mesylate is especially useful for the treatment of humans suffering from psychotic disorders or Parkinson's disease.

The following examples are only intended to further illustrate the invention, in more detail, and therefore these examples are not deemed to restrict the scope of the invention in any way.

### Example 1. Preparation of bifeprunox mesylate.

### Example 1a. Preparation of N-(5-chloro-2-hydroxyphenyl)acetamide.

143.6 g (1 mole) of 2-amino-4-chlorphenol was suspended in 550 ml of methyl t-butyl ether under mild nitrogen purge. The mixture was heated to reflux until the material was dissolved. In 40 minutes 112.3 g of acetic anhydride was added. After the addition the mixture was cooled to 20-25°C in one hour. After stirring for an additional hour the mixture was cooled to 0-5°C under stirring and kept on this temperature for an additional hour. The product was filtered off, washed with 200 ml of methyl *t*-butyl ether twice and dried at 50°C and 100 mbar under a gentle nitrogen stream till dry. Yield about 92%.

### Example 1b. Preparation of N-(5-chloro-2-hydroxy-3-nitrophenyl)acetamide.

224.5 g of sulphuric acid (50% w/w) was dissolved in 300 ml of water and cooled to 25°C while stirring under a mild nitrogen purge. 185.1 g (1 mole) of N-(5-chloro-2-hydroxyphenyl)acetamide prepared according to Example 1a was added to the diluted sulphuric acid and mixed intensively. 4 ml of nitric acid 65% w/w was added to the foam formed on top of the reaction mixture at low stirring speed. The stirring speed was increased and 75 ml of nitric acid 65% w/w was added in 45 minutes, while maintaining the temperature between 23 and 26 °C. The mixture was stirred vigorously for an additional 1 hour at 23 -26°C.Then the mixture was cooled to 0-5°C and vigorously stirred at this temperature for 1 hour. The solid was filtered off quickly, washed three times with 300 ml of cold water, sucked for at lea st 30 minutes and dried at 50°C and 100 mbar under a gentle nitrogen stream till dry.

The crude product was suspended in 2000 ml 96% ethanol, heated till reflux and refluxed under stirring for about 15 minutes until a clear solution was obtained. The solution was cooled to 25-30°C in about 1 hour, while stirring slowly, further cooled to 0-5°C and stirred at this temperature for an additional hour. The solid was filtered off, washed twice with 250 ml of cold 96% ethanol, and dried at 50 °C and 100 mbar under a gentle nitrogen stream till dry. Yield about 78%.

### Example 1c. Preparation of 6-amino-4-chloro-2-nitrophenol.

230.6 g (1 mole) of N-(5-chloro-2-hydroxy-3-nitrophenyl)acetamide prepared according to Example 1b was suspended in a mixture of 950 ml of wat er and 100 ml of 2-propanol under a mild nitrogen purge. 345 ml of 36% w/w hydrochloric acid was added followed by 25 ml of water. The mixture was heated to reflux in about 30 °C, while vigorously stirring and refluxed for 2 hours. The mixture was cooled to 0-5°C in about one hour and stirred for an additional hour at 0-5°C. The solid was filtered off, washed twice with 250 ml of water, and dried at 50°C and 100 mbar under a gentle nitrogen stream till dry. Yield about 91 %.

### Example 1d. Preparation of 5-chforo-7-nitro-2(3H)-benzoxazolone.

188.6 g (1 mole) of 6-amino-4-chloro-2-nitrophenol prepared according to Example 1c was suspended in 1000 ml of ethyl acetate under mild nitrogen purge and the optional present water was removed by azeotropic distillation of 250 ml of the solvent. The mixture was cooled to 20-25°C and 224 g of carbonyldiimidazole was added as a slurry in 650 ml of ethyl acetate. An additional 100 ml of ethyl acetate was added and the mixture was vigorously stirred during two hours, without the application of cooling. 1000 ml of water was added and the mixture was stirred for 15 minutes. 1450-1500 ml of ethyl acetate was distilled off at about 200 mBar and about 50°C. The mixture was cooled to 0-5°C, 225 ml of 36% HCl was added and the mixture ws cooled again to 0-5°C and stirred at this temperature for 15 minutes. The solid was filtered off, washed with 400 ml of 1 N HCl, washed twice with 500 ml of cold water and once with 500 ml of cold water/ethanol (4/1), and dried at 50 °C and 100 mbar under a gentle nitrogen stream till dry. Yield about 99%.

### Example 1e. Preparation of 7-amino-2(3H)-benzoxazolone.

107.5 g (1 mole) of 5-chloro-7-nitro-2(3H)-benzoxazolone prepared according to Example 1d was suspended in 1000 ml of ethanol. 9.25 g of Pd/C 5% and 50 ml of ethanol were added and the mixture was hydrogenated at 4 bar hydrogen pressure for four to six hours at 60-65°C while vigorously stirring. When the hydrogenation was complete, the mixture was cooled to 45°C and filtered over Hyflo®. The Hyflo® was washed twice with 175 ml of methanol. 500 ml of solvent was distilled off under reduced pressure at 50°C, followed by addition of 250 ml of water and removal of 300 ml of solvent was by distillation under reduced pressure at 50 °C. The last procedure was repeated twice and finally 250 ml of water was added and 400 ml of solvent was distilled of. The resulting mixture was cooled to 0-5°C in about one hour. The solid was filtered off and, washed three times with 125 ml of cold water, and dried at 50°C and 100 mbar under a gentle nitrogen stream till dry. Yield about 94%.

### Example 1f. Preparation of 3-[[bis(2-hydroxyethyl)amino]methyl]-1,1'-biphenyl

A mixture was prepared of 123.4 g of diethanolamine, 100 ml of water and 100 ml of methylethylketone (MEK) and 500 ml of methyl *t*-butyl ether while stirring under a mild nitrogen purge 124.75 g of 3-(bromomethyl)-1,1'-biphenyl was added together with 750 ml of methyl *t*-butyl ether. The mixture was heated to reflux and refluxed for 18 hours, followed by cooling till room temperature. Thereafter the mixture was washed once with 375 ml of 2N NaOH and four times with 375 ml of water. The combined 2N NaOH and water layers were extracted with 750 ml of methyl *t*-butyl ether. The combined methyl *t*-butyl ether layers were washed with 250 ml of water followed by distillation of as much methyl *t*-butyl ether as possible from the organic layer. 1350 ml of methylethylketone was added and 600 ml of solvent was distilled of at atmospheric pressure. The solution was cooled to room temperature and stored for use in the next step. Yield based on quantitative assay 97%.

### Example 1g. Preparation of bifeprunox mesylate (crude)

A solution of 128.9 g of 3-[[bis(2-hydroxyethyl)amino]methyl]-1,1'-biphenyl in approximately 750 ml of methylethylketone prepared according to Example 1f was stirred under mild nitrogen purge. In a separate vessel 202 g of methanesulfonic anhydride was dissolved in in 600 ml of methylethylketone at 10-20°C. To the solution of 3-[[bis(2-hydroxyethyl)amino]methyl]-1,1'-biphenyl in methylethylketone 212.8 g of triethylamine was added and 60 ml of methylethylketone. The solution of methanesulfonic anhydride was added in about 45-60 minutes to the 3-[[bis(2-hydroxyethyl)amino]methyl]-1,1'-biphenyl/triethylamine solution, while maintaining the temperature below 10°C. 60 ml of methylethylketone was added and the mixture was stirred for another 15 minutes, followed by drop wise addition of 109.7 g of methanesulfonic acid and addition of 60 ml of methylethylketone in order to rinse the addition vessel.

71.3 g of 7-amino-2(3H)-benzoxazolone, prepared according to Example 1e was suspended in 100 ml of methylethylketone and added to the reaction mixture followed by 60 ml of methylethylketone. The reaction mixture was heated to reflux and refluxed during 20-24 hours. After 20-24 hours of reflux 48 ml of water was added and the mixture was refluxed again for 1 hour. 420 ml of methylethylketone was added and 490 ml of methylethylketone/water was distilled of. This last step was rep eated three times. 46.1 g of methanesulphonic acid was added, the mixture was refluxed for an additional hour and cooled down to room temperature in 1 hour. The mixture was further cooled down to 0-5°C and stirred at this temperature for another hour. The solid was filtered off and, washed twice with 75 ml of cold methylethylketone, and dried at 50°C and 100 mbar under a gentle nitrogen stream till dry. Yield about 76%.

### Example 2. Preparation of polymorphic form α of bifeprunox mesylate in 2-propanol.

10.06 g of bifeprunox mesylate crude prepared as described Example 1g was suspended in a mixture of 200 ml of 2-propanol and 40 ml of water under nitrogen purge. The suspension was heated until reflux and cooled down to room temperature in 120 minutes under stirring. The formed suspension was further cooled down under stirring to 0°C and stirred at this temperature for a further 120 minutes. The crystals were filtered of and dried at 50°C and 100 mbar.

### Example 3. Preparation of polymorphic form α of bifeprunox mesylate in acetronitrile.

50 g of bifeprunox mesylate prepared crude as described in Example 1g was suspended in a mixture of 875 ml of acetonitrile and 250 ml of water under nitrogen purge. 375 ml of acetonitrile was added and the reaction mixture was heated till reflux. 500 ml of solvent was distilled off and 500 ml of acetonitrile were added and this procedure was repeated for a second time. After distilling another 500 ml of solvent the mixture was cooled down to room temperature in 120 minutes. The mixture was further cooled down to 5-0°C and stirred for 120 minutes at this temperature. The formed crystals were filtered off and washed twice with acetonitrile. The isolated crystals were dried at 50°C and 100 mbar under a mild nitrogen purge. Yield 85.6%.

### Example 4. Preparation of polymorphic form γ of bifeprunox mesylate

To a suspension of 12.50 g of bifeprunox mesylate (crude) prepared as described in Example 1g in 150 ml of methylethylketone (MEK) 75 ml of a 5% NaHCO₃ solution was added in about 10-15 min. After 5-10 minutes of stirring the suspension was filtered over Hyflo® or Celite® into another vessel where the layers were separated. The water layer was extracted with 125 and 75 ml of methylethylketone. The methylethylketone layers were combined and washed with a mixture of 50 ml of water and 10 ml of ethanol 96%.

The methylethylketone layer was filtered through a 1 µm filter into a clean vessel, after which the filter was rinsed with 25 ml of methylethylketone. Methylethylketone was distilled off until a volume of about 130 ml was reached, 200 ml of methylethylketone was added and again methylethylketone was distilled off to reach a volume of 175 ml.

Then a solution of 3.00 grams of methane sulphonic acid in 50 ml of methylethylketone was added in about 30 min. After cooling to 5 °C and stirring for 1½ hours at this temperature the product was filtered off and washed twice with 50 ml of cold methylethylketone. After drying at 50°C and 100 mbar under a mild nitrogen purge the gamma polymorph of bifeprunox mesylate was yielded in about 80%.

### Example 5. Preparation of a 10 mg capsule formulation of polymorphic form α of bifeprunox mesylate.

2.227 kg of lactose was sieved and filled into a high shear mixer. 125 g of bifeprunox mesylate in its polymorphic form α was sieved and added. The composition was mixed with a high shear mixer (e.g. Collette Gral 10 or Collette Gal 75) until it was homogenous (approximately 4 minutes). 24 g of a disintegrant (e.g. sodium starch glycolate USP-NF such as Primojel®) and 24 g of a lubricant (e.g. sodium stearyl fumarate such as PRUV®) were added and the composition was mixed again until it was homogenous (approximately 1 minute). The powder was filled into capsules size 0, 240 mg per capsule by means of a capsule filling machine (e.g. Zanasi LZ 64 or Zanasi RM63 plug filler). Approximately 10,000 filled capsules were obtained.

### Example 6. Preparation of a 10 mg tablet formulation of bifeprunox mesylate polymorphic form α.

Tablets with a strength of 10 mg were prepared according to the following procedures (required quantities are given in Table 8). One third of the given amount of lactose monohydrate was sieved and filled into a high shear mixer and mixed during 5 minutes. The required amount of milled bifeprunox mesylate in its polymorphic form α was added to the mixture, together with 0.100 kg sodium starch glycolate, type A, 2.32 kg microcrystalline cellulose and the remainder of the lactulose monohydrate. The composition was mixed with a high shear mixer (e. g. Collette Gral 10 or Collette Gal 75) until- it was homogenous (approximately 10 minutes). The required amount of a sodium stearyl fumarate (such as PRUV®), sieved through a 0.42 mm sieve was added and the composition was mixed again until it was homogenous (approximately 5 minutes). The final product was compressed with 300 MPa into tablets. The product was coated using 15% m/m of the indicated Opadry II HP water suspension to 3.5 % of the core weight.

**Table 8. Required amount of active ingredient and auxiliary materials large scale production of 10 mg bifeprunox mesylate tablets.**

| Components | Per batch of 83333 10 mg tablets (in kg) |
|---|---|
| Core components | |
| Bifeprunox mesylate (milled) | 1.041 |
| Lactose monohydrate | 16.33 |
| Microcrystalline cellulose | 2.32 |
| Sodium starch glycolate, type A | 0.100 |
| Sodium stearyl fumarate | 0.200 |
| Coating components | |
| Opadry II HP beige 85F27126 | 0.700 |
| Purified water | 3.968 |

### Example 7. Analytical methods.

XRPD patterns were measured on a diffractometer using monochromatic CuKα radiation (tube voltage 40 kV, tube current 40 mA) at room temperature. IR spectra were recorded on a Fourier transform IR spectrometer in attenuated total reflectance (silicon crystal) with a spectral resolution of 2 cm⁻¹ using a mercury cadmium telluride detector.

Melting points were determined on a DSC apparatus as onset temperatures of the melting endotherm using 40 µL aluminum crucibles with a pierced lid. Temperature program: heating from 25 °C up to 300 °C with 10 K min⁻¹. N₂ atmosphere at a flow of 80 mL min⁻¹.

The solid state ¹³C NMR spectra were obtained using the cross-polarisation magic-angle spinning (CP/MAS) accessory on a Bruker AM300 instrument (contact time of 4 ms, recycle delay 3 s, spectral width 30kHz, ¹H 90° pulse of 6 µs, spinning rate about 8.5kHz. A standard 4 mm Bruker CP/MAS probe was used. Chemical shifts are referred to glycine (δ_{c}=176.03 ppm for the C=O resonance).

Analyses for the alpha and the delta crystal forms were carried out at the Bijvoet Centre for Biomolecular Research, Utrecht University. Analysis of the gamma crystal form was carried out in Peter Jones' lab in Institute of Inorganic and Analytical Chemistry, University of Braunsweig.

Crystals of the alpha form appeared under the microscope as block-shaped, those of the gamma crystal form were plate- or rod-shaped, whereas crystals of the delta crystal form looked block-shaped with rounded edges.

For each crystal form, a crystal was transferred into the cold nitrogen stream on a rotating anode X-ray diffractometer. The structures were solved by automated direct methods. Hydrogen atoms bonded to nitrogen were located on an electron -density map and their coordinates were included as parameters in the refinement. Other hydrogen atoms were included in the refinement on calculated positions riding on their carrier atoms. All non-hydrogen atoms were refined with anisotropic atomic displacement parameters. Hydrogen atoms were given fixed displacement factors, related to those of their carrier atoms.

## Claims

1. A crystalline polymorph of 7-[4-([1,1'-biphenyl]-3-ylmethyl)-1-piperazinyl]-2(3H)-benzoxazolone monomethanesulfonate, exhibiting an X-ray powder diffraction pattern having characteristic peaks expressed in degrees 2 θ at approximately 7.0, 9.3, 10.0, 12.5, 15.4, 16.7, 17.2, 17.4, 17.7, 18.7, 21.3, 22.2, 25.2, 27.2, 28.3, 28.8 and 30.1.

2. A crystalline polymorph of 7-[4-([1,1'-biphenyl]-3-ylmethyl)-1-piperazinyl]-2(3H)-benzoxazolone monomethanesulfonate according to claim 1, **characterized by** an X-ray powder diffraction (=XRPD) pattern substantially as shown in Figure 1.

3. A crystalline polymorph of 7-[4,([1,1'biphenyl],3-ylmethyl)-1-piperazinyl]-2(3H)-benzoxazolone monomethanesulfonate according to claim 1, having a melting point at approximately 277 °C.

4. A crystalline polymorph of 7-[4-([1,1'-biphenyl]-3-ylmethyl)-1-piperazinyl]-2(3H)-benzoxazolone monomethanesulfonate according to claim 1, **characterized by** a complete DSC trace substantially as shown in Figure 2

5. A crystalline polymorph of 7-[4-([1,1'-biphenyl]-3-ylmethyl)-1-piperazinyl]-2(3H)-benzoxazolone monomethanesulfonate according to claim 1 exhibiting an attenuated total reflectance infrared spectrum having characteristic absorption bands expressed in reciprocal centimeters at approximately 1764, 1217, 795, 746 and 694.

6. A crystalline polymorph of 7-[4-([1,1'-biphenyl]-3-ylmethyl)-1-piperazinyl]-2(3H)-benzoxazolone monomethanesulfonate according to claim 5, exhibiting an attenuated total reflectance infrared spectrum having characte ristic absorption bands expressed in reciprocal centimeters at approximately 1764, 1636, 1284, 1217, 809, 795, 746, 694, 663 and 509.

7. A crystalline polymorph of 7,[4,([1,1'-biphenyl],3-ylmethyl)-1-piperazinyl]-2(3H)-benzoxazolone monomethanesulfonate according to claim 6, **characterized by** a complete IR spectrum substantially as shown in Figure 3

8. A crystalline polymorph of 7-[4-([1,1'-biphenyl]-3-ylmethyl)-1-piperazinyl]-2(3H)-benzoxazolone monomethanesulfonate according to claim 1, exhibiting ¹³C solid state NMR chemical shifts expressed relative to glycine (δc =176.03 for the C=O resonance) at approximately 40.4, 48.7, 56.5, 106.8 and 137.7 ppm.

9. A crystalline polymorph of 7-[4-([1,1'-biphenyl]-3-ylmethyl)-1-piperazinyl]-2(3H)-benzoxazolone monomethanesulfonate according to claim 8, exhibiting ¹³C solid state NMR chemical shifts expressed relative to glycine (δ_{c} =176.03 for the C=O resonance) at approximately 40.4, 48.7, 50.3, 56.5, 106.8, 110.7, 124.9, 126.9, 127.8, 129.2, 130.8, 134.2, 137.7, 141.6, and 153.8 ppm.

10. A crystalline polymorph of 7-[4-([1,1'-biphenyl]-3-ylmethyl)-1-piperazinyl]-2(3H)-benzoxazolone monomethanesulfonate according to claim 9, **characterized by** a complete ¹³C solid state NMR chemical substantially as shown in Figure 4.

11. A crystalline polymorph of 7-[4-([1,1'-biphenyl]-3-ylmethyl)-1-piperazinyl]-2(3H)-benzoxazolone monomethanesulfonate according to claim 1, exhibiting a single crystal X-ray crystallographic analysis at 150K with (a) crystal parameters that are approximately equal to the following:
| | |
|---|---|
| • Cell dimensions | a = 9.283 Å |
| | b = 10.737 Å |
| | c = 12.690 Å |
| | α = 98.553° |
| | β = 93.749° |
| | γ = 116.097° |
| • Crystal system | triclinic |
| • Space group | P-1 |
| • Molecules/unit cell | 2 |
| • Density (g/cm³) | 1.481 |
and (b) atomic positions of all atoms relative to the origin of the unit cell as recited in Table 5

12. A crystalline polymorph of 7-[4-([1,1'-biphenyl]-3-ylmethyl)-1-piperazinyl]-2(3H)-benzoxazolone monomethanesulfonate according to claim 11, **characterized by** a single crystal X-ray diffraction substantially as shown in Figure 5.

13. 7-[4-([1,1'-biphenyl]-3-ylmethyl)-1-piperazinyl]-2(3H)-benzoxazolone monomethanesulfonate in which at least about 50 weight % of the compound is in the crystalline polymorphic form according to any of claims 1-12.

14. The composition according to claim 13, **characterized in that** at least about 60 weight % of the compound is in the crystalline polymorphic form according to any of claims 1-8.

15. The composition according to claim 14, **characterized in that** at least about 80 weight % of the compound is the crystalline polymorphic form according to any of claims 1-8.

16. The composition according to claim 15, **characterized in that** at least about 90 weight % of the compound is in the crystalline polymorphic form according to claims any of 1-8.

17. The composition according to claim 16, **characterized in that** at least 95 weight % of the compound is in the crystalline polymorphic form according to claims 1-8.

18. A process for the preparation of the crystalline polymorph according to any of claims 1-12, which comprises crystallizing 7-[4-([1,1'-biphenyl]-3-ylmethyl)-1-piperazinyl]-2(3H)-benzoxazolone monomethanesulfonate from a solution thereof in an organic solvent or a mixture of an organic solvent with water.

19. A process for the preparation of the crystalline polymorph according to any of claims 1-12, which comprises recrystallizing the γ or δ polymorph of 7-[4-([1,1'-biphenyl]-3-ylmethyl)-1-piperazinyl]-2(3H)-benzoxazolone monomethanesulfonate or a mixture of the two polymorphs from a solution thereof in an organic solvent or a mixture of an organic solvent with water.

20. A process according to claim 18 or 19, wherein said organic solvent is selected from the group consisting of 2-propanol and acetonitrile and preferably is acetonitrile.

21. A pharmaceutical composition comprising an effective amount of the crystalline polymorph according to any of claims 1-12 and at least one pharmaceutically acceptable excipient.

22. A pharmaceutical composition, comprising an effective amount of 7-[4-([1,1'-biphenyl]-3-ylmethyl)-1-piperazinyl]-2(3H)-benzoxazolone monomethanesulphonate according to any of claims 1- 12 and a mixture of lactulose
monohydrate, microcrystalline lactose, microcrystalline cellulose, sodium starch glycolate type A, sodium stearyl fumarate and optionally colloidal anhydrous silica.

23. A pharmaceutical composition according to claim 22, comprising an effective amount of the crystalline polymorph according to any of claims 1- 12, between 70% and 90% w/w of lactose monohydrate, between 10% and 15% w/w of microcrystalline cellulose, between 0.3% and 0.7% w/w of sodium starch glycolate type A, between 0.6% and 1.3% w/w of sodium stearyl fumarate and optionally between 0.05% and 0.5% w/w of colloidal anhydrous silica.

24. A pharmaceutical composition according to claims 22-23, comprising an effective amount of the crystalline polymorph according to any of claims 1-12.

25. The crystalline polymorph according to any of claims 1-12 for use in medicine,

26. The crystalline polymorph according to any of claims 1-12 for use in the treatment of CNS disorders, comprising administering an effective amount of the crystalline polymorph.

27. The crystalline polymorph according to claim 26, wherein said CNS disorder is a psychotic disorder or Parkinson's disease.

28. The crystalline polymorph according to claim 26, wherein said CNS disorder is schizophrenia.

## Patentansprüche

1. Kristalliner Polymorph von 7-[4-([1,1'-Biphenyl]-3-ylmethyl)-1-piperazinyl]-2(3H)-benzoxazolon-Monomethansulfonat, das ein Röntgenpulverdiffraktionsmuster aufweist, das, in Graden ausgedrückt, charakteristische Peaks 2 θ bei etwa 7,0, 9,3, 10,0, 12,5, 15,4, 16,7, 17,2, 17,4, 17,7, 18,7, 21,3, 22,2, 25,2, 27,2, 28,3, 28,8 und 30,1 hat.

2. Kristalliner Polymorph von 7-[4-([1,1'-Biphenyl]-3-ylmethyl)-1-piperazinyl]-2(3H)-benzoxazolon-Monomethansulfonat nach Anspruch 1, **gekennzeichnet durch** ein Röntgenpulverdiffraktions-(=XRPD)muster im Wesentlichen wie in Fig. 1 gezeigt.

3. Kristalliner Polymorph von 7-[4-([1,1'-Biphenyl]-3-ylmethyl)-1-piperazinyl]-2(3H)-benzoxazolon-Monomethansulfonat nach Anspruch 1 mit einem Schmelzpunkt bei etwa 277°C.

4. Kristalliner Polymorph von 7-[4-([1,1'-Biphenyl]-3-ylmethyl)-1-piperazinyl]-2(3H)-benzoxazolon-Monomethansulfonat nach Anspruch 1, **gekennzeichnet durch** eine vollständige DSC-Kurve im Wesentlichen wie in Fig. 2 gezeigt.

5. Kristalliner Polymorph von 7-[4-([1,1'-Biphenyl]-3-ylmethyl)-1-piperazinyl]-2(3H)-benzoxazolon-Monomethansulfonat nach Anspruch 1, welcher ein Infrarotspektrum mit abgeschwächter Totalreflexion aufweist, das charakteristische Absorptionsbanden hat, ausgedrückt in reziproken Zentimetern bei etwa 1764, 1217, 795, 746 und 694.

6. Kristalliner Polymorph von 7-[4-([1,1'-Biphenyl]-3-ylmethyl)-1-piperazinyl]-2(3H)-benzoxazolon-Monomethansulfonat nach Anspruch 5, welcher ein Infrarotspektrum mit abgeschwächter Totalreflexion aufweist, das charakteristische Absorptionsbanden hat, ausgedrückt in reziproken Zentimetern bei etwa 1764, 1636, 1284, 1217, 809, 795, 746, 694, 663 und 509.

7. Kristalliner Polymorph von 7-[4-([1,1'-Biphenyl]-3-ylmethyl)-1-piperazinyl]-2(3H)-benzoxazolon-Monomethansulfonat nach Anspruch 6, **gekennzeichnet durch** ein komplettes IR-Spektrum im Wesentlichen wie in Fig. 3 gezeigt.

8. Kristalliner Polymorph von 7-[4-([1,1'-Biphenyl]-3-ylmethyl)-1-piperazinyl]-2(3H)-benzoxazolon-Monomethansulfonat nach Anspruch 1, welcher im ¹³C-Festphasen-NMR chemische Verschiebungen, ausgedrückt im Verhältnis zu Glycin (δ_{c} = 176,03 für die C=O-Resonanz), bei etwa 40,4, 48,7, 56,5, 106,8 und 137,7 ppm aufweist.

9. Kristalliner Polymorph von 7-[4-([1,1'-Biphenyl]-3-ylmethyl)-1-piperazinyl]-2(3H)-benzoxazolon-Monomethansulfonat nach Anspruch 8, welcher im ¹³C-Festphasen-NMR chemische Verschiebungen, ausgedrückt im Verhältnis zu Glycin (δ_{c} = 176,03 für die C=O-Resonanz), bei etwa 40,4, 48,7, 50,3, 56,5, 106,8, 110,7, 124,9, 126,9, 127,8, 129,2, 130,8, 134,2, 137,7, 141,6 und 153,8 ppm aufweist.

10. Kristalliner Polymorph von 7-[4-([1,1'-Biphenyl]-3-ylmethyl)-1-piperazinyl]-2(3H)-benzoxazolon-Monomethansulfonat nach Anspruch 9, **gekennzeichnet durch** eine vollständige chemische Verschiebung im ¹³C-Festphasen-NMR, im Wesentlichen wie in Fig. 4 gezeigt.

11. Kristalliner Polymorph von 7-[4-([1,1'-Biphenyl]-3-ylmethyl)-1-piperazinyl]-2(3H)-benzoxazolon-Monomethansulfonat nach Anspruch 1, welcher eine Einkristall-Röntgenkristallographie-Analyse bei 15K aufweist mit (a) Kristall-Parametern, die etwa gleich dem Folgenden sind:
| | |
|---|---|
| • Zellabmessungen | a = 9,283 Å |
| | b = 10,737 Å |
| | c = 12,690 Å |
| | α = 98,553° |
| | β = 93,749° |
| | γ = 116,097° |
| • Kristallsystem | triklin |
| • Raumgruppe | P-1 |
| • Moleküle/Elementarzelle | 2 |
| • Dichte (g/cm³) | 1,481 |
und (b) Atompositionen aller Atome in Bezug auf den Ursprung der Elementarzelle wie in Tabelle 5 angeführt.

12. Kristalliner Polymorph von 7-[4-([1,1'-Biphenyl]-3-ylmethyl)-1-piperazinyl]-2(3H)-benzoxazolon-Monomethansulfonat nach Anspruch 11, **gekennzeichnet durch** eine Einkristallröntgendiffraktion im Wesentlichen wie in Fig. 5 gezeigt.

13. 7-[4-([1,1'-Biphenyl]-3-ylmethyl)-1-piperazinyl]-2(3H)-benzoxazolon-Monomethansulfonat, bei welchem mindestens 50 Gew.-% der Verbindung in kristallin polymorpher Form gemäß einem der Ansprüche 1-12 vorliegen.

14. Zusammensetzung nach Anspruch 13, **dadurch gekennzeichnet, dass** mindestens etwa 60 Gew.-% der Verbindung in der kristallin polymorphen Form nach einem der Ansprüche 1-8 vorliegen.

15. Zusammensetzung nach Anspruch 14, **dadurch gekennzeichnet, dass** mindestens etwa 80 Gew.-% der Verbindung in der kristallin polymorphen Form nach einem der Ansprüche 1-8 vorliegen.

16. Zusammensetzung nach Anspruch 15, **dadurch gekennzeichnet, dass** mindestens etwa 90 Gew.-% der Verbindung in der kristallin polymorphen Form nach einem der Ansprüche 1-8 vorliegen.

17. Zusammensetzung nach Anspruch 16, **dadurch gekennzeichnet, dass** mindestens 95 Gew.-% der Verbindung in der kristallin polymorphen Form nach einem der Ansprüche 1-8 vorliegen.

18. Verfahren zur Herstellung des kristallinen Polymorphs nach einem der Ansprüche 1-12, welches das Kristallisieren von 7-[4-([1,1'-Biphenyl]-3-ylmethyl)-1-piperazinyl]-2(3H)-benzoxazolon-Monomethansulfonat aus einer Lösung davon in einem organischen Lösungsmittel oder einer Mischung von einem organischen Lösungsmittel mit Wasser umfasst.

19. Verfahren zur Herstellung des kristallinen Polymorphs nach einem der Ansprüche 1-12, welches das Umkristallisieren des γ-oder δ-Polymorphs von 7-[4-([1,1'-Biphenyl]-3-ylmethyl)-1-piperazinyl]-2(3H)-benzoxazolon-Monomethansulfonat oder einer Mischung der beiden Polymorphen aus einer Lösung davon in einem organischen Lösungsmittel oder einer Mischung von einem organischen Lösungsmittel mit Wasser umfasst.

20. Verfahren nach Anspruch 18 oder 19, wobei das organische Lösungsmittel ausgewählt ist aus der Gruppe bestehend aus 2-Propanol und Acetonitril und vorzugsweise Acetonitril ist.

21. Pharmazeutische Zusammensetzung, umfassend eine wirksame Menge des kristallinen Polymorphs nach einem der Ansprüche 1-12 und mindestens einen pharmazeutisch akzeptablen Exzipienten.

22. Pharmazeutische Zusammensetzung, umfassend eine wirksame Menge von 7-[4-([1,1'-Biphenyl]-3-ylmethyl)-1-piperazinyl]-2(3H)-benzoxazolon-Monomethansulfonat nach einem der Ansprüche 1-12 und eine Mischung aus Lactulose-Monohydrat, mikrokristalliner Lactose, mikrokristalliner Cellulose, Natriumstärkeglykolat Typ A, Natriumstearylfumarat und gegebenenfalls kolloidalem wasserfreiem Siliziumdioxid.

23. Pharmazeutische Zusammensetzung nach Anspruch 22, umfassend eine wirksame Menge des kristallinen Polymorphs nach einem der Ansprüche 1-12, zwischen 70% und 90% Gew./Gew. Lactose-Monohydrat, zwischen 10% und 15% Gew./Gew. mikrokristalline Cellulose, zwischen 0,3% und 0,7% Gew./Gew. Natriumstärkeglykolat Typ A, zwischen 0,6% und 1,3% Gew./Gew. Natriumstearylfumarat und gegebenenfalls zwischen 0,05% und 0,5% Gew./Gew. kolloidales wasserfreies Siliziumdioxid.

24. Pharmazeutische Zusammensetzung nach den Ansprüchen 22-23, umfassend eine wirksame Menge des kristallinen Polymorphs nach einem der Ansprüche 1-12.

25. Kristalliner Polymorph nach einem der Ansprüche 1-12 zur Verwendung in der Medizin.

26. Kristalliner Polymorph nach einem der Ansprüche 1-12 zur Verwendung bei der Behandlung von Erkrankungen des ZNS, umfassend das Verabreichen einer wirksamen Menge des kristallinen Polymorphs.

27. Kristalliner Polymorph nach Anspruch 26, wobei die ZNS-Erkrankung eine psychotische Erkrankung oder Morbus Parkinson ist.

28. Kristalliner Polymorph nach Anspruch 26, wobei die Erkrankung des ZNS Schizophrenie ist.

## Revendications

1. Polymorphe cristallin de monométhanesulfonate de 7-[4-([1,1'-biphényl]-3-ylméthyl)-1-pipérazinyl]-2(3H)-benzoxazolone exhibant un schéma de diffraction de rayons X sur poudre ayant des pics caractéristiques exprimés en degrés 2 θ à environ 7,0, 9,3, 10,0, 12,5, 15,4, 16,7, 17,2, 17,4, 17,7, 18,7, 21,3, 22,2, 25,2, 27,2, 28,3, 28,8 et 30,1.

2. Polymorphe cristallin de monométhanesulfonate de 7-[4-([1,1'-biphényl]-3-ylméthyl)-1-pipérazinyl]-2(3H)-benzoxazolone selon la revendication 1, **caractérisé par** un schéma de diffraction de rayons X sur poudre (XRPD) en grande partie comme illustré sur la figure 1.

3. Polymorphe cristallin de monométhanesulfonate de 7-[4-([1,1'-biphényl]-3-ylméthyl)-1-pipérazinyl]-2(3H)-benzoxazolone selon la revendication 1, possédant un point de fusion d'environ 277 °C.

4. Polymorphe cristallin de monométhanesulfonate de 7-[4-([1,1'-biphényl]-3-ylméthyl)-1-pipérazinyl]-2(3H)-benzoxazolone selon la revendication 1, **caractérisé par** un tracé DSC complet en grande partie comme illustré sur la figure 2.

5. Polymorphe cristallin de monométhanesulfonate de 7-[4-([1,1'-biphényl]-3-ylméthyl)-1-pipérazinyl]-2(3H)-benzoxazolone selon la revendication 1, exhibant un spectre infrarouge de réflexion totale atténuée ayant des bandes d'absorption caractéristiques exprimées en centimètres réciproques à environ 1764, 1217, 795, 746 et 694.

6. Polymorphe cristallin de monométhanesulfonate de 7-[4-([1,1'-biphényl]-3-ylméthyl)-1-pipérazinyl]-2(3H)-benzoxazolone selon la revendication 5, exhibant un spectre infrarouge de réflexion totale atténuée ayant des bandes d'absorption caractéristiques exprimées en centimètres réciproques à environ 1764, 1636, 1284, 1217, 809, 795, 746, 694, 663 et 509.

7. Polymorphe cristallin de monométhanesulfonate de 7-[4-([1,1'-biphényl]-3-ylméthyl)-1-pipérazinyl]-2(3H)-benzoxazolone selon la revendication 6, **caractérisé par** un spectre IR complet en grande partie comme illustré sur la figure 3.

8. Polymorphe cristallin de monométhanesulfonate de 7-[4-([1,1'-biphényl]-3-ylméthyl)-1-pipérazinyl]-2(3H)-benzoxazolone selon la revendication 1, exhibant des déplacements chimiques par RMN du ¹³C à l'état solide exprimés par rapport à la glycine (δ_{c}= 176,03 pour la résonance de C=O) à environ 40,4, 48,7, 56,5, 106,8 et 137,7 ppm.

9. Polymorphe cristallin de monométhanesulfonate de 7-[4-([1,1'-biphényl]-3-ylméthyl)-1-pipérazinyl]-2(3H)-benzoxazolone selon la revendication 8, exhibant des déplacements chimiques par RMN du ¹³C à l'état solide exprimés par rapport à la glycine δ_{c} = 176,03 pour la résonance de C=O) à environ 40,4, 48,7, 50,3, 56,5, 106,8, 110,7, 124,9, 126,9, 127,8, 129,2, 130,8, 134,2, 137,7, 141,6 et 153,8 ppm.

10. Polymorphe cristallin de monométhanesulfonate de 7-[4-([1,1'-biphényl]-3-ylméthyl)-1-pipérazinyl]-2(3H)-benzoxazolone selon la revendication 9, **caractérisé par** des déplacements chimiques par RMN du ¹³C à l'état solide en grande partie comme illustré sur la figure 4.

11. Polymorphe cristallin de monométhanesulfonate de 7-[4-([1,1'-biphényl]-3-ylméthyl)-1-pipérazinyl]-2(3H)-benzoxazolone selon la revendication 1, exhibant une analyse cristallographique aux rayons X d'un cristal unique à 150 K avec (a) des paramètres cristallins qui sont approximativement équivalents à ce qui suit :
| | |
|---|---|
| • Dimensions de la cellule | a = 9,283 Å |
| | b = 10,737 Å |
| | c = 12,690 Å |
| | α = 98,553° |
| | β = 93,749° |
| | γ = 116,097° |
| • Système cristallin | triclinique |
| • Groupe d'espace | P-1 |
| • Molécules/cellule unitaire | 2 |
| • Densité (g/cm³) | 1,481 |
et (b) des positions atomiques de tous les atomes par rapport à la position d'origine de la cellule unitaire comme décrit dans le tableau 5.

12. Polymorphe cristallin de monométhanesulfonate de 7-[4-([1,1'-biphényl]-3-ylméthyl)-3-pipérazinyl]-2(3H)-benzoxazolone selon la revendication 11, **caractérisé par** une diffraction de rayons X d'un cristal unique en grande partie comme illustré sur la figure 5.

13. Monométhanesulfonate de 7-[4-([1,1'-biphényl]-3-ylméthyl)-1-pipérazinyl]-2(3H)-benzoxazolone dans lequel au moins environ 50 % en poids du composé est sous la forme polymorphe cristalline selon l'une quelconque des revendications 1 à 12.

14. Composition selon la revendication 13, **caractérisée en ce qu'**au moins environ 60 % en poids du composé est sous la forme polymorphe cristalline selon l'une quelconque des revendications 1 à 8.

15. Composition selon la revendication 14, **caractérisée en ce qu'**au moins environ 80 % en poids du composé est sous la forme polymorphe cristalline selon l'une quelconque des revendications 1 à 8.

16. Composition selon la revendication 15, **caractérisée en ce qu'**au moins environ 90 % en poids du composé est sous la forme polymorphe cristalline selon l'une quelconque des revendications 1 à 8.

17. Composition selon la revendication 16, **caractérisée en ce qu'**au moins environ 95 % en poids du composé est sous la forme polymorphe cristalline selon l'une quelconque des revendications 1 à 8.

18. Procédé de préparation du polymorphe cristallin selon l'une quelconque des revendications 1 à 12, qui comprend la cristallisation de monométhanesulfonate de 7-[4-([1,1'-biphényl]-3-ylméthyl)-1-pipérazinyl]-2(3H)-benzoxazolone à partir d'une solution de celui-ci dans un solvant organique ou un mélange d'un solvant organique et d'eau.

19. Procédé de préparation du polymorphe cristallin selon l'une quelconque des revendications 1 à 12, qui comprend la cristallisation du polymorphe γ ou δ de monométhanesulfonate de 7-[4-([1,1'-biphényl]-3-ylméthyl)-1-pipérazinyl]-2(3H)-benzoxazolone ou d'un mélange des deux polymorphes à partir d'une solution de celui-ci dans un solvant organique ou un mélange d'un solvant organique et d'eau.

20. Procédé selon la revendication 18 ou 19, dans lequel ledit solvant organique est choisi dans le groupe constitué par le 2-propanol et l'acétonitrile, et de préférence est l'acétonitrile.

21. Composition pharmaceutique comprenant une quantité efficace du polymorphe cristallin selon l'une quelconque des revendications 1 à 12 et au moins un excipient pharmaceutiquement acceptable.

22. Composition pharmaceutique comprenant une quantité efficace de monométhanesulfonate de 7-[4-([1,1'-biphényl]-3-ylméthyl)-1-pipérazinyl]-2(3H)-benzoxazolone selon l'une quelconque des revendications 1 à 12 et un mélange de monohydrate de lactose, lactose microcristallin, cellulose microcristalline, glycolate d'amidon de sodium de type A, fumarate de stéaryle sodique et facultativement silice anhydre colloïdale.

23. Composition pharmaceutique selon la revendication 22, comprenant une quantité efficace du polymorphe cristallin selon l'une quelconque des revendications 1 à 12, de 70 % à 90 % en poids de monohydrate de lactose, de 10 % à 15 % en poids de cellulose microcristalline, de 0,3 % à 0,7 % en poids de glycolate d'amidon de sodium de type A, de 0,6 % à 1,3 % en poids de fumarate de stéaryle sodique et facultativement de 0,05 % à 0,5 % en poids de silice anhydre colloïdale..

24. Composition pharmaceutique selon les revendications 22 et 23, comprenant une quantité efficace du polymorphe cristallin selon l'une quelconque des revendications 1 à 12.

25. Polymorphe cristallin selon l'une quelconque des revendications 1 à 12, pour une utilisation en médecine.

26. Polymorphe cristallin selon l'une quelconque des revendications 1 à 12, pour une utilisation dans le traitement de troubles du SNC, comprenant l'administration d'une quantité efficace du polymorphe cristallin.

27. Polymorphe cristallin selon la revendication 26, dans lequel ledit trouble du SNC est un trouble psychotique ou la maladie de Parkinson.

28. Polymorphe cristallin selon la revendication 26, dans lequel ledit trouble du CNS est la schizophrénie.
